# EUROPÄISCHE PATENTSCHRIFT

(11) **EP 3 374 012 B1**
(45) Veröffentlichungstag und Bekanntmachung des Hinweises auf die Patenterteilung: **04.10.2023**
(21) Anmeldenummer: 16788720.7
(22) Anmeldetag: 02.11.2016
(51) Int. Cl.: A61M 16/00, G01N 27/10

(54) **BEATMUNGSGERÄT**
VENTILATOR
APPAREIL DE VENTILATION

(30) Priorität: 13.11.2015 DE 102015119636
(43) Veröffentlichungstag der Anmeldung: 19.09.2018
(73) Patentinhaber: Kagan, Eugen, 53859 Niederkassel Lülsdorf (DE)
(72) Erfinder: Kagan, Eugen, 53859 Niederkassel Lülsdorf (DE)
(74) Vertreter: Wagner Albiger & Partner Patentanwälte mbB
(86) Internationale Anmeldenummer: PCT/EP2016/076413
(87) Internationale Veröffentlichungsnummer: WO 2017/080884

(56) Entgegenhaltungen:
- DE-B3- 10 259 988
- US-A1- 2002 098 120
- US-A1- 2006 201 509
- US-A1- 2007 225 612
- US-A1- 2010 139 659
- US-A1- 2011 209 707

## Beschreibung

Die vorliegende Erfindung betrifft ein Beatmungsgerät mit einem Gasleitungssystem zur Versorgung eines Patienten mit Atemgas.

Eine derartige Beatmungsvorrichtung ist beispielsweise aus der DE 10 2006 055 779 B3 bekannt. Sie umfasst Zuleitungen, insbesondere für Sauerstoff und Druckluft, die in der Beatmungsvorrichtung in einer vorgegebenen Weise gemischt werden, sowie eine Leitung, mit der das fertig gemischte, temperierte und auf einem vorgegebenen Druckniveau gehaltene Atemgas dem Patienten zuführbar ist. Eine derartige Beatmungsvorrichtung weist typischerweise mindestens einen Sauerstoffsensor auf zur Überwachung des Sauerstoffgehalts des Atemgases. Der Sauerstoffsensor kann dabei im Bereich der zum Patienten geführten Leitung, im Bereich einer Mischkammer oder in einem anderen Bereich des Leitungssystems angeordnet sein. Es können auch mehrere Sauerstoffsensoren vorgesehen sein.

Aus der US 2006/0201509 A ist ein Drucklufttauchgerät für den Tauchsport bekannt, in das mehrere, insbesondere redundante Sauerstoffsensoren einsetzbar sind. US2010/139659 A1 betrifft eine Vorrichtung und ein Verfahren zur Steuerung der Sauerstoffdosierung eines Beatmungsgerätes,

In Beatmungsgeräten für medizinische Zwecke kommen typischerweise galvanische, auch "elektrochemisch" genannte Sauerstoffsensoren zum Einsatz, bei denen beispielsweise der Sauerstoff durch eine Membran in eine mit einem Elektrolyt gefüllte Kammer diffundiert. In dieser Kammer wird der Sauerstoff an einer Goldelektrode reduziert und eine Bleielektrode gleichzeitig oxidiert. Dadurch fließt ein zur Sauerstoffkonzentration proportionaler Strom, welcher als Messsignal ausgewertet werden kann. Es ist eine Vielzahl auf diesem Prinzip beruhender Messzellen bekannt, wobei verschiedene Elektrolyte und Elektrodenmaterialien zum Einsatz kommen können. Gemeinsam ist diesen Methoden, dass die Bildung von Oxid an der einen Elektrode zu einem allmählichen Verbrauch der Elektrode führt. Ein derartiger galvanischer Sauerstoffsensor hat demnach eine begrenzte Lebensdauer. Er hat jedoch den Vorteil, dass er sehr robust und damit auch für den Betrieb in einem mobilen Beatmungsgerät geeignet ist.

Ebenfalls geeignet für medizinische Beatmungsgeräte sind paramagnetische Sauerstoffsensoren, die die paramagnetischen Eigenschaften des Sauerstoffs ausnutzen. Dabei kann entweder eine feinmechanische Vorrichtung vorgesehen sein, die durch den in das Magnetfeld gezogenen Sauerstoff bewegt wird, oder es wird direkt oder indirekt die Geschwindigkeit eines Sauerstoffstromes in einem Messkanal bestimmt.

Die paramagnetische Sauerstoffmessung ist sehr genau und nicht verbrauchend. Ein paramagnetischer Sauerstoffsensor ist somit langlebig. Er ist jedoch, insbesondere wenn feinmechanische Bauteile eingesetzt werden, verhältnismäßig empfindlich und somit ungeeignet für einen mobilen Betrieb der Beatmungsvorrichtung.

Es ist eine Aufgabe der vorliegenden Erfindung, ein Beatmungsgerät mit einem Gasleitungssystem zur Versorgung eines Patienten mit Atemgas anzugeben, das vielseitig einsetzbar und gleichzeitig sehr zuverlässig ist.

Diese Aufgabe wird gelöst durch den Gegenstand des unabhängigen Patentanspruchs. Weitere Ausführungsformen sind Gegenstand der Unteransprüche.

Gemäß einem Aspekt der Erfindung wird ein Beatmungsgerät angegeben, aufweisend ein Gasleitungssystem zur Versorgung eines Patienten mit Atemgas, wobei zumindest eine Überwachungsvorrichtung zur Überwachung des Sauerstoffgehalts des Atemgases vorgesehen ist, wobei die Überwachungsvorrichtung zumindest eine Aufnahme für einen Sauerstoffsensor aufweist. In die Aufnahme ist wahlweise ein galvanischer Sauerstoffsensor oder ein paramagnetischer Sauerstoffsensor einsetzbar.

Unter einer Überwachungsvorrichtung zur Überwachung des Sauerstoffgehalts des Atemgases wird hier eine Einrichtung verstanden, die Mittel zum direkten oder indirekten Messen des Sauerstoffgehalts und Mittel zur Auswertung bzw. Verarbeitung dieser Messwerte aufweist, also beispielsweise einen Sensor mit einer entsprechenden Steuereinrichtung.

Bei einem derartigen Beatmungsgerät sind somit die Sauerstoffsensoren gegeneinander austauschbar, das heißt, es ist eine Aufnahme vorgesehen, in die beide Sauerstoffsensoren passen, jedoch nicht gemeinsam. Vielmehr kann vor der Inbetriebnahme des Beatmungsgerätes der jeweils passende Sauerstoffsensor ausgewählt und in die Aufnahme eingesetzt werden.

Das hat den Vorteil, dass für einen mobilen Betrieb des Beatmungsgerätes der robuste galvanische Sensor ausgewählt werden kann, während für einen stationären Betrieb des Beatmungsgerätes der sehr genaue und langlebige paramagnetische Sensor ausgewählt werden kann.

Durch die Austauschbarkeit der Sensoren ist es somit möglich, für jeden Einsatzzweck des Beatmungsgerätes den passenden Sensortyp einzusetzen. Die Vor- und Nachteile der beiden Sensortypen können somit optimal berücksichtigt werden.

Da nur entweder der eine oder der andere Sensor in die Aufnahme eingesetzt werden kann, wird vermieden, dass aus Gründen der Bequemlichkeit beide Sensoren im Gerät belassen werden, so dass der empfindliche paramagnetische Sensor beim mobilen Betrieb des Beatmungsgerätes mechanischen Belastungen ausgesetzt wird. Ferner wird auch die Wahrscheinlichkeit dafür verringert, dass der galvanische Sensor versehentlich oder aus Gründen der Bequemlichkeit im stationären Betrieb im Beatmungsgerät belassen und verwendet wird, so dass sich seine Lebensdauer reduziert. Ferner wird durch das Vorsehen einer Aufnahme für nur einen einzigen Sensor Bauraum und Gewicht gespart, was insbesondere für mobile Einsatzzwecke vorteilhaft ist.

Im stationären Betrieb hat die Verwendung des paramagnetischen Sensors ferner den Vorteil, dass dieser besonders genau ist, so dass die Zusammensetzung des Atemgases mit hoher Genauigkeit geregelt werden kann.

Das Beatmungsgerät ist somit besonders ökonomisch betreibbar und besonders langlebig und zuverlässig.

Gemäß einer Ausführungsform ist der Sauerstoffsensor mittels eines Befestigungsbauteils in der Aufnahme befestigbar. Dabei kann das Befestigungsbauteil gewissermaßen als Adapter ausgebildet sein, mittels dessen sowohl der eine als auch der andere Sensortyp in der Aufnahme befestigt werden kann.

Beispielsweise kann das Befestigungsbauteil ein Gewinde zur Verbindung mit einem galvanischen Sauerstoffsensor aufweisen. Der galvanische Sauerstoffsensor weist dann beispielsweise an einem Ende das passende Gegenstück zu diesem Gewinde auf und kann somit in das Befestigungsbauteil eingeschraubt werden. Das Befestigungsbauteil verankert dann den Sauerstoffsensor in der Aufnahme.

Zudem kann das Befestigungsbauteil eine Anzahl von Bohrungen zur Verbindung mit einem paramagnetischen Sauerstoffsensor mittels einer Anzahl von Schrauben aufweisen. Gegenstücke zu diesen Bohrungen können beispielsweise an dem paramagnetischen Sensor vorhanden sein, so dass der paramagnetische Sensor und das Befestigungsbauteil miteinander verschraubt werden können.

Denkbar sind auch andere Arten der Befestigung zwischen dem Befestigungsbauteil und den Sensoren, beispielsweise Verrastungen oder ein Bajonettverschluss.

Die Verwendung eines separaten Befestigungsbauteils hat den Vorteil, dass das Einsetzen eines Sensors in die Aufnahme besonders einfach ist, da der Sensor außerhalb der Aufnahme mit dem Befestigungsbauteil verbunden werden kann. Die möglicherweise etwas Geschicklichkeit erfordernde Verbindung zwischen dem Sensor und dem Befestigungsbauteil kann somit bequem außerhalb der Aufnahme erfolgen. Anschließend erfolgt das Einsetzen des Sensors mit dem Befestigungsbauteil in die Aufnahme. Dazu sind das Befestigungsbauteil und die Aufnahme derart ausgestaltet, dass die Verbindung des Befestigungsbauteils mit der Aufnahme besonders einfach erfolgen kann, beispielsweise durch eine Rastverbindung.

Zur elektrischen Verbindung des Sauerstoffsensors mit dem Beatmungsgerät ist ein erster, dem Beatmungsgerät zugeordneter Anschluss und zwei zweite Anschlüsse im Bereich der Aufnahme vorgesehen, wobei der eine zweite Anschluss zur Verbindung mit einem galvanischen Sauerstoffsensor vorgesehen ist und der andere zweite Anschluss zur Verbindung mit einem paramagnetischen Sauerstoffsensor.

Zum Auslesen des Sensorsignals ist somit eine verzweigte Verbindung zwischen dem Sensor und dem Beatmungsgerät vorgesehen. In der Aufnahme stehen somit unterschiedliche Anschlüsse für die beiden Sensortypen zur Verfügung. Das hat den Vorteil, dass für die Sensoren Standardbauteile verwendet werden können, bei denen die Ausgestaltungen der Anschlüsse und ihre Lage vorgegeben sind. Typischerweise weisen galvanische und paramagnetische Sauerstoffsensoren nämlich nicht nur unterschiedliche Typen von Anschlüssen auf, sondern diese sind auch bei dem in die Aufnahme eingesetztem Sensor an unterschiedlichen Stellen zugänglich. Durch die Verwendung einer verzweigten Verbindung zwischen dem Beatmungsgerät und dem Sensor wird dem Rechnung getragen: Für jeden der beiden Sensortypen steht an der passenden Stelle der passende zweite Anschluss zur Verfügung.

Typischerweise sind die zweiten Anschlüsse dabei derart im Bereich der Aufnahme angeordnet, dass im Falle eines in die Aufnahme eingesetzten galvanischen Sensors dieser lediglich mit dem einen zweiten Anschluss verbindbar ist, während bei einem in die Aufnahme eingesetzten paramagnetischen Sensor dieser lediglich mit dem anderen zweiten Anschluss verbindbar ist.

Dies hat auch den Vorteil, dass je nach Ausgestaltung der Verbindung bereits anhand der Tatsache, welcher der zweiten Anschlüsse mit einem Sensor belegt ist, auf den in die Aufnahme eingesetzten Sensortyp geschlossen werden kann. Eine Identifikation des in die Aufnahme eingesetzten Sensors kann dann durch die Feststellung erfolgen, welcher zweite Anschluss belegt ist.

Gemäß einer Ausführungsform ist der Sauerstoffsensor mit dem Beatmungsgerät über ein Y-Kabel verbindbar, d. h. die verzweigte Verbindung ist durch ein Y-Kabel realisiert, dass an seinem verzweigten Ende unterschiedliche Stecker oder Buchsentypen aufweisen kann und mit seinem anderen Ende beispielsweise auf eine Platine des Beatmungsgerätes aufsteckbar und dadurch mit einer Steuereinheit des Beatmungsgeräts verbindbar ist.

Gemäß einer Ausführungsform erkennt eine Steuereinheit des Beatmungsgerätes, welcher Sauerstoffsensor in die Aufnahme eingesetzt ist. Die Erkennung kann auf verschiedene Weise erfolgen, beispielsweise anhand des belegten zweiten Anschlusses wie oben beschrieben.

Es ist aber auch möglich, dass der Sauerstoffsensor eine Elektronikeinheit aufweist, mittels der er mit einer Steuereinheit des Beatmungsgerätes kommunizieren kann. In diesem Fall kann der Sauerstoffsensor selbst sich bei der Steuereinheit des Beatmungsgerätes anmelden.

Gemäß einer Ausführungsform ist die Aufnahme durch einen Verschlussdeckel verschließbar, wenn kein Sauerstoffsensor eingesetzt ist. Der Verschlussdeckel hat insbesondere den Vorteil, dass er die Aufnahme vor Verschmutzung und Beschädigungen schützt. Er kann derart ausgebildet sein, dass er die Aufnahme auch bei eingesetztem Sensor verschließen kann.

Das Beatmungsgerät kann insbesondere sowohl für den mobilen als auch für den stationären Betrieb ausgelegt sein. Das hat den Vorteil, dass das Beatmungsgerät besonders flexibel einsetzbar ist.

Dazu weist es insbesondere passende Anschlüsse für Versorgungsleitungen auf. Für den stationären Betrieb werden dabei Anschlüsse für Druckgasflaschen oder fest eingebaute Leitungen einer Druckgasversorgungsanlage eines Krankenhauses vorgesehen. Im mobilen Betrieb ist typischerweise der Anschluss einer Druckgasflasche und/oder der Einsatz einer Turbine, die Umgebungsluft ansaugt, vorgesehen. Ein Beatmungsgerät, das sowohl für den mobilen als auch für den stationären Betrieb ausgelegt ist, weist typischerweise auch die Möglichkeit auf, verschiedene Betriebsmodi auszuwählen, insbesondere einen mobilen und einen stationären Betriebsmodus.

Ausführungsformen der Erfindungen werden im Folgenden anhand von Figuren näher beschrieben. Darin zeigen
- Figur 1: schematisch ein Beatmungsgerät gemäß einer Ausführungsform der Erfindung;
- Figur 2: Details des Beatmungsgerätes gemäß Figur 1 ohne eingesetzten Sauerstoffsensor;
- Figur 3: schematisch die Aufnahme des Beatmungsgerätes mit eingesetztem galvanischen Sensor;
- Figur 4: schematisch die Aufnahme des Beatmungsgerätes gemäß Figur 1 mit eingesetztem paramagnetischen Sauerstoffsensor;
- Figur 5: schematisch den mit einem Befestigungsbauteil des Beatmungsgerätes gemäß Figur 1 verbundenen galvanischen Sauerstoffsensor;
- Figur 6: schematisch den mit dem Befestigungsbauteil verbundenen galvanischen Sensor gemäß Figur 5 aus einer anderen Perspektive;
- Figur 7: schematisch den mit dem Befestigungsbauteil des Beatmungsgerätes gemäß Figur 1 verbundenen paramagnetischen Sauerstoffsensor und
- Figur 8: den mit dem Befestigungsbauteil verbundenen paramagnetischen Sensor gemäß Figur 7 aus einer anderen Perspektive.

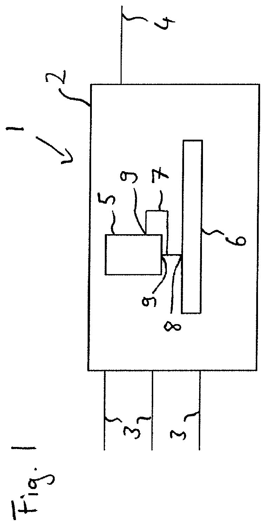

Figur 1 zeigt ein Beatmungsgerät 1 zur Versorgung eines Patienten mit Atemgas. Das Beatmungsgerät 1 weist ein Gehäuse 2 auf, das nicht näher dargestellte Anschlüsse für Zuleitungen 3 für Druckgas umfasst. Als Druckgas kommen insbesondere Sauerstoff und Druckluft in Frage, wobei die Druckluft einer Druckgasflasche, einer Druckgasversorgungsanlage eines Krankenhauses oder einer Turbine entnommen werden kann. Der Sauerstoff kann insbesondere einer Druckgasflasche oder einer Druckgasversorgungsanlage eines Krankenhauses entnommen werden.

Das Beatmungsgerät 1 weist ferner eine Leitung 4 auf, über die dem Patienten Atemgas zugeführt wird. Das Atemgas wird innerhalb des Gehäuses 2 des Beatmungsgerätes 1 aus den über die Zuleitungen 3 zugeführten Gasen gemischt. Dabei können insbesondere die Zusammensetzung, der Druck und die Temperatur des Atemgases in einer vorgegebenen Weise geregelt werden.

In dem Gehäuse 2 ist eine Aufnahme 5 für einen Sauerstoffsensor vorgesehen. Zum Auslesen des Sensorsignals kann der in die Aufnahme 5 eingesetzte Sensor elektrisch mit einer Steuereinheit 6 des Beatmungsgerätes 1 verbunden werden. Die Verbindung erfolgt über ein Y-Kabel 7, das mit einem Ende mit einem dem Beatmungsgerät 2 bzw. seiner Steuereinheit 6 zugeordneten ersten Anschluss 8 verbunden wird.

Im Bereich der Aufnahme 5 sind zwei zweite Anschlüsse vorgesehen, die durch die beiden verzweigten Enden des Y-Kabels 7 zur Verfügung gestellt werden.

In die Aufnahme 5 ist wahlweise entweder ein galvanischer Sauerstoffsensor oder ein paramagnetischer Sauerstoffsensor einsetzbar. Ist ein galvanischer Sauerstoffsensor eingesetzt, so wird dieser mit dem einen zweiten Anschluss verbunden. Ist ein paramagnetischer Sauerstoffsensor eingesetzt, so wird dieser mit dem anderen zweiten Anschluss 9 verbunden.

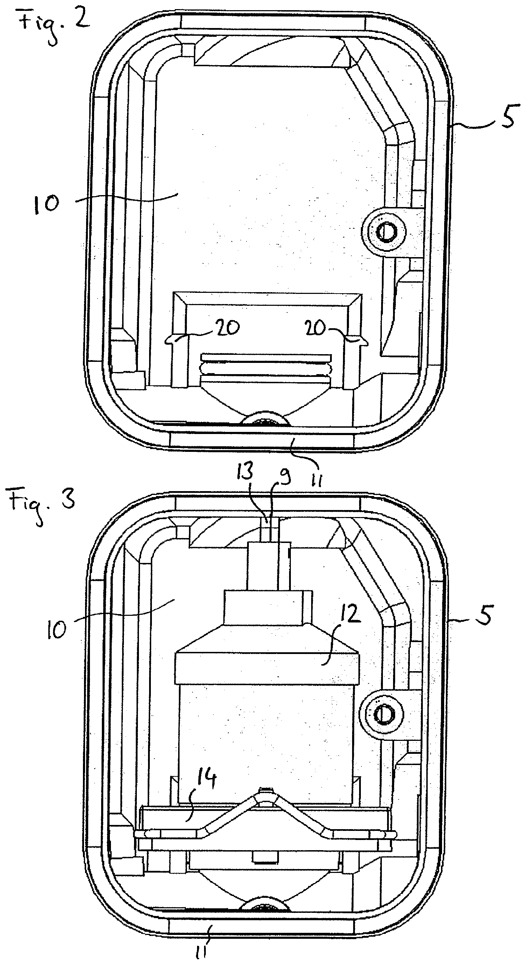

Figur 2 zeigt einen Schnitt durch die Aufnahme 5 im Gehäuse 2 des Beatmungsgerätes 1. Dabei ist erkennbar, dass die Aufnahme 5 einen Hohlraum 10 zur Aufnahme eines Sauerstoffsensors aufweist, der an einer Seite durch einen Verschlussdeckel 11 verschlossen ist. Der Verschlussdeckel 11 weist innerhalb des Hohlraums 10 gelegene Rastnasen 20 auf.

Figur 3 zeigt die Aufnahme 5 mit dem darin eingesetzten galvanischen Sensor 12. Der galvanische Sensor 12 ist innerhalb des Hohlraums 10 angeordnet. An einem Ende ist er mit einem Befestigungsbauteil 14 verbunden, und zwar über ein in dieser Darstellung nicht sichtbares Gewinde. Das Befestigungsbauteil 14 ist über die Rastnasen 20 mit dem Verschlussdeckel 11 verbunden.

An seinem anderen Ende weist der galvanische Sensor 12 einen Anschluss 13 zum Auslesen des Sensorsignals auf. Dieser Anschluss 13 kann beispielsweise als Klinkenstecker ausgebildet sein. Er ist mit dem zweiten Anschluss 9 verbunden, der durch das eine verzweigte Ende des Y-Kabels 7 gemäß Figur 1 zur Verfügung gestellt wird.

Zum Einsetzen des galvanischen Sensors 12 in die Aufnahme 5 wird folgendermaßen vorgegangen:
Zunächst wird der galvanische Sensor 12 außerhalb des Hohlraums 10 mit dem Befestigungsbauteil 14 dadurch verbunden, dass er in dieses eingeschraubt wird. Anschließend wird das Befestigungsbauteil 14 mit dem galvanischen Sensor 12 in den Hohlraum 10 eingeführt, wobei der Anschluss 13 des galvanischen Sensors 12 mit dem zweiten Anschluss 9 verbunden wird. Ferner wird der Verschlussdeckel 11 aufgesetzt, wobei die Rastnasen 20 an dem Befestigungsbauteil 14 einrasten. Die Verbindung des Befestigungsbauteils 14 mit dem Verschlussdeckel 11 kann vor oder nach dem Einsetzen des galvanischen Sensors 12 in den Hohlraum 10 erfolgen.

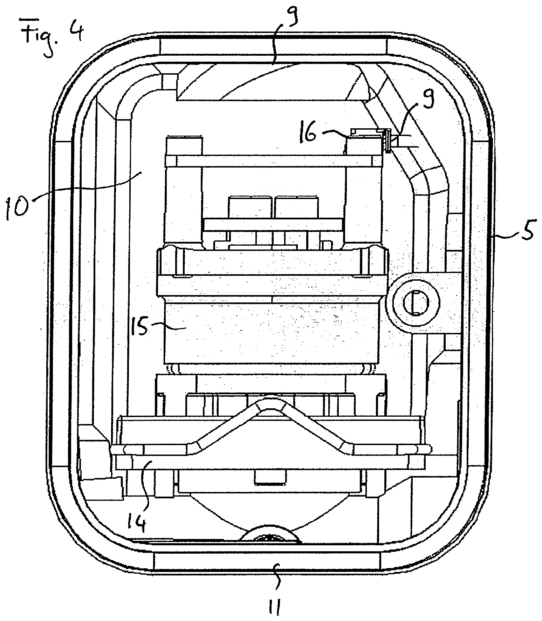

Figur 4 zeigt die Aufnahme 5 des Beatmungsgerätes 1 mit einem darin eingesetzten paramagnetischen Sensor 15. Der paramagnetische Sensor 15 ist an seinem einen Ende ebenfalls mit dem Befestigungsbauteil 14 verbunden, und zwar über eine Anzahl von in entsprechende Bohrungen am Befestigungsbauteil 14 und am paramagnetischen Sensor 15 eingesetzte Schrauben. An seinem anderen Ende weist der paramagnetische Sensor 15 einen Anschluss 16 auf, der mit dem anderen zweiten Anschluss 9 verbunden ist und die elektrische Verbindung zwischen dem paramagnetischen Sensor 15 und der Steuereinheit 6 des Beatmungsgerätes 1 herstellt.

Zum Einsetzen des paramagnetischen Sensors 15 in den Hohlraum 10 wird analog wie zu Figur 3 beschrieben vorgegangen: zunächst wird der paramagnetische Sensor 15 außerhalb des Hohlraums 10 mit dem Befestigungsbauteil 14 verschraubt. Anschließend wird entweder der Verschlussdeckel 11 am Befestigungsbauteil 14 verrastet und dieses zusammen mit dem Sensor in den Hohlraum 10 eingeführt oder es wird zunächst lediglich das Befestigungsbauteil 14 mit dem Sensor 15 in den Hohlraum eingeführt und die elektrische Verbindung zwischen dem Anschluss 16 und dem anderen zweiten Anschluss 9 hergestellt und anschließend wird der Verschlussdeckel 11 an dem Befestigungsbauteil 14 verrastet.

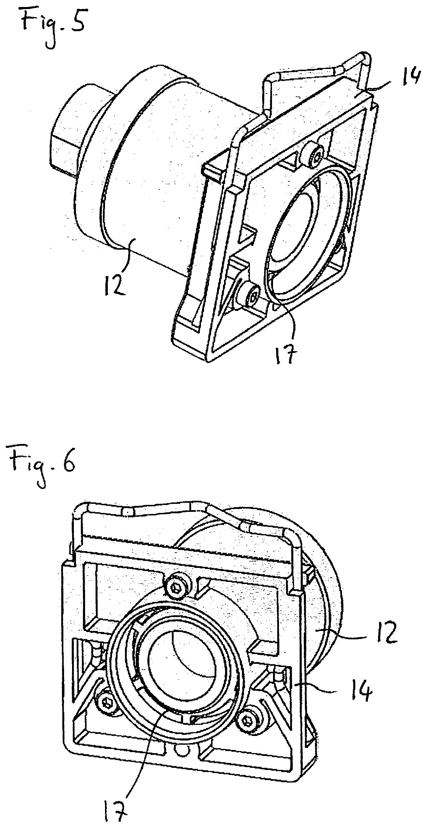

Die Figuren 5 und 6 zeigen perspektivische Ansichten des galvanischen Sensors 12, der mit dem Befestigungsbauteil 14 verbunden ist. In dieser Ansicht ist erkennbar, dass der galvanische Sensor 12 im Bereich seines einen Endes ein Außengewinde aufweist, zu dem ein Innengewinde 17 des Befestigungsbauteils 14 korrespondiert.

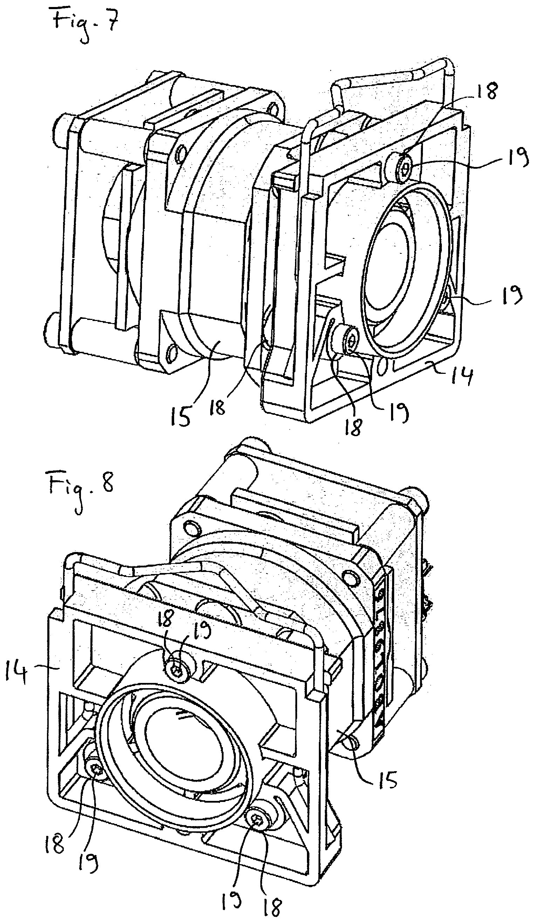

Die Figuren 7 und 8 zeigen perspektivische Ansichten des mit dem Befestigungsbauteil 14 verbundenen paramagnetischen Sensors 15. In dieser Ansicht ist erkennbar, dass sowohl das Befestigungsbauteil 14 als auch der paramagnetische Sensor 15 bzw. sein Gehäuse jeweils drei Bohrungen 18 aufweisen, in die Schrauben 19 zur Verbindung des Befestigungsbauteils 14 mit dem paramagnetischen Sensor 15 einsetzbar sind.

Die Verbindung sowohl des galvanischen Sensors 12 als auch des paramagnetischen Sensors 15 mit dem Befestigungsbauteil 14 kann auf verschiedene Weisen erfolgen. Es ist auch denkbar, den paramagnetischen Sensor über ein Gewinde mit dem Befestigungsbauteil 14 zu verschrauben oder eine Anzahl von Schrauben zur Verbindung des galvanischen Sensors 12 mit dem Befestigungsbauteil 14 vorzusehen. Darüber hinaus wären auch beispielsweise Rastverbindungen denkbar. Zu beachten ist, dass die Art der Verbindung zwischen dem Befestigungsbauteil 14 und dem Sensor 12, 15 die Orientierung des Sensors 12, 15 in dem Hohlraum 10 festlegen kann. Dies muss im Hinblick auf die Platzierung der zweiten Anschlüsse 9 berücksichtigt werden.

In der gezeigten Ausführungsform ist der Anschluss 13 des galvanischen Sensors 12, wie in Figur 3 erkennbar, rotationssymmetrisch ausgestaltet. Die Winkelstellung des Sensors 12 innerhalb des Hohlraums ist somit beliebig. Somit ist eine Verbindung des galvanischen Sensors 12 mit dem Befestigungsbauteil 14 über ein Schraubgewinde unproblematisch.

Der paramagnetische Sensor 15 muss jedoch bei dem gezeigten Ausführungsbeispiel innerhalb des Hohlraums 10 derart ausgerichtet sein, dass sein Anschluss 16 an dem anderen zweiten Anschluss 9 zu liegen kommt. Er ist nicht rotationssymmetrisch, seine Orientierung innerhalb des Hohlraums 10 ist somit nicht beliebig. Somit ist eine Verbindung zwischen dem Befestigungsbauteil 14 und dem paramagnetischen Sensor 15, wie sie in den Figuren 7 und 8 gezeigt ist, mittels einer Anzahl von Schrauben 19 vorteilhaft, weil sie die Orientierung des Befestigungsbauteils 14 zu dem paramagnetischen Sensor 15 festlegt. Wird der derart mit dem Befestigungsbauteil 14 verbundene paramagnetische Sensor 15 dann in die Aufnahme 5 eingeführt, so kommt der Anschluss 16 automatisch an dem anderen zweiten Anschluss 9 zu liegen.

### Bezugszeichenliste

- 1: Beatmungsgerät
- 2: Gehäuse
- 3: Zuleitung
- 4: Leitung
- 5: Aufnahme
- 6: Steuereinheit
- 7: Y-Kabel
- 8: erster Anschluss
- 9: zweiter Anschluss
- 10: Hohlraum
- 11: Verschlussdeckel
- 12: galvanischer Sensor
- 13: Anschluss
- 14: Befestigungsbauteil
- 15: paramagnetischer Sensor
- 16: Anschluss
- 17: Innengewinde
- 18: Bohrung
- 19: Schraube
- 20: Rastnase

## Patentansprüche

1. Beatmungsgerät (1), aufweisend ein Gasleitungssystem zur Versorgung eines Patienten mit Atemgas, wobei zumindest eine Überwachungsvorrichtung zur Überwachung des Sauerstoffgehalts des Atemgases vorgesehen ist, wobei die Überwachungsvorrichtung zumindest eine Aufnahme (5) für einen Sauerstoffsensor (12, 15) aufweist,
**dadurch gekennzeichnet, dass**
in die Aufnahme (5) wahlweise ein galvanischer Sauerstoffsensor (12) oder ein paramagnetischer Sauerstoffsensor (15) einsetzbar ist, wobei die beiden Sauerstoffsensoren nicht gemeinsam in die Aufnahme passen,
wobei zur elektrischen Verbindung des Sauerstoffsensors (12, 15) mit dem Beatmungsgerät (1) ein erster, dem Beatmungsgerät (1) zugeordneter Anschluss (8) und zwei zweite Anschlüsse (9) im Bereich der Aufnahme (5) vorgesehen sind, wobei der eine zweite Anschluss (9) zur Verbindung mit einem galvanischen Sauerstoffsensor (12) vorgesehen ist und der andere zweite Anschluss (9) zur Verbindung mit einem paramagnetischen Sauerstoffsensor (15).

2. Beatmungsgerät (1) nach Anspruch 1,
**dadurch gekennzeichnet, dass**
der Sauerstoffsensor (12, 15) mittels eines Befestigungsbauteils (14) in der Aufnahme (5) befestigbar ist.

3. Beatmungsgerät (1) nach Anspruch 2,
**dadurch gekennzeichnet, dass**
das Befestigungsbauteil (14) ein Gewinde (17) zur Verbindung mit einem galvanischen Sauerstoffsensor (12) aufweist.

4. Beatmungsgerät (1) nach Anspruch 2 oder 3,
**dadurch gekennzeichnet, dass**
das Befestigungsbauteil (14) eine Anzahl von Bohrungen (18) zur Verbindung mit einem paramagnetischen Sauerstoffsensor (15) mittels einer Anzahl von Schrauben (19) aufweist.

5. Beatmungsgerät (1) nach einem der Ansprüche 1 bis 4,
**dadurch gekennzeichnet, dass**
die zweiten Anschlüsse (9) derart im Bereich der Aufnahme (5) angeordnet sind, dass im Falle eines in die Aufnahme (5) eingesetzten galvanischen Sensors (12) dieser lediglich mit dem einen zweiten Anschluss (9) verbindbar ist, während im Falle eines in die Aufnahme (5) eingesetzten paramagnetischen Sauerstoffsensors (15) dieser lediglich mit dem anderen zweiten Anschluss (9) verbindbar ist.

6. Beatmungsgerät (1) nach einem der Ansprüche 1 bis 5,
**dadurch gekennzeichnet, dass**
der Sauerstoffsensor (12, 15) mit dem Beatmungsgerät (1) über ein Y-Kabel (7) verbindbar ist.

7. Beatmungsgerät (1) nach einem der Ansprüche 1 bis 6,
**dadurch gekennzeichnet, dass**
eine Steuereinheit (6) des Beatmungsgerätes (1) erkennt, welcher Sauerstoffsensor (12, 15) in die Aufnahme (5) eingesetzt ist.

8. Beatmungsgerät (1) nach einem der Ansprüche 1 bis 7,
**dadurch gekennzeichnet, dass**
der Sauerstoffsensor (12, 15) eine Elektronikeinheit aufweist, mittels der er mit einer Steuereinheit (6) des Beatmungsgeräts (1) kommunizieren kann.

9. Beatmungsgerät (1) nach einem der Ansprüche 1 bis 8,
**dadurch gekennzeichnet, dass**
die Aufnahme (5) durch einen Verschlussdeckel (11) verschließbar ist, wenn kein Sauerstoffsensor (12, 15) eingesetzt ist.

10. Beatmungsgerät (1) nach einem der Ansprüche 1 bis 9,
**dadurch gekennzeichnet, dass**
das Beatmungsgerät (1) sowohl für den mobilen als auch für den stationären Betrieb ausgelegt ist.

## Claims

1. Ventilator (1) having a gas guidance system for supplying a patient with breathing gas, wherein at least one monitoring device for monitoring the oxygen content of the breathing gas is provided, wherein the monitoring device has at least one receptacle (5) for an oxygen sensor (12, 15),
**characterised in that**
optionally a galvanic oxygen sensor (12) or a paramagnetic oxygen sensor (15) can be inserted into the receptacle (5), wherein the two oxygen sensors do not fit together into the receptacle,
wherein for the electrical connection of the oxygen sensor (12, 15) to the ventilator (1) a first terminal (8) assigned to the ventilator (1) and two second terminals (9) are provided in the region of the receptacle (5), wherein the one second terminal (9) is provided for connection to a galvanic oxygen sensor (12) and the other second terminal (9) is provided for connection to a paramagnetic oxygen sensor (15).

2. Ventilator (1) according to claim 1,
**characterised in that**
the oxygen sensor (12, 15) can be fastened in the receptacle (5) by means of a fastening component (14).

3. Ventilator (1) according to claim 2,
**characterised in that**
the fastening component (14) has a threading (17) for connection to a galvanic oxygen sensor (12).

4. Ventilator (1) according to claim 2 or 3,
**characterised in that**
the fastening component (14) has a number of bores (18) for connection to a paramagnetic oxygen sensor (15) by means of a number of screws (19).

5. Ventilator (1) according to any of claims 1 to 4,
**characterised in that**
the second terminals (9) are arranged in the region of the receptacle (5) such that in the case of a galvanic sensor (12) being inserted into the receptacle (5) this is connectable only to the one second terminal (9), while in the case of a paramagnetic oxygen sensor (15) being inserted into the receptacle (5) this is connectable only to the other second terminal (9).

6. Ventilator (1) according to any of claims 1 to 5,
**characterised in that**
the oxygen sensor (12, 15) is connectable to the ventilator (1) by way of a y-cable (7).

7. Ventilator (1) according to any of claims 1 to 6,
**characterised in that**
a control unit (6) of the ventilator (1) recognises which oxygen sensor (12, 15) is inserted into the receptacle (5).

8. Ventilator (1) according to any of claims 1 to 7,
**characterised in that**
the oxygen sensor (12, 15) has an electronic unit by means of which it is able to communicate with a control unit (6) of the ventilator (1).

9. Ventilator (1) according to any of claims 1 to 8,
**characterised in that**
the receptacle (5) is closable by means of a closure lid (11) when no oxygen sensor (12, 15) is inserted.

10. Ventilator (1) according to any of claims 1 to 9,
**characterised in that**
the ventilator (1) is designed both for mobile operation and for stationary operation.

## Revendications

1. Respirateur (1), présentant un système de conduites de gaz pour alimenter un patient en gaz respiratoire, dans lequel au moins un dispositif de surveillance est prévu pour surveiller la teneur en oxygène du gaz respiratoire, dans lequel le dispositif de surveillance présente au moins un logement (5) pour un capteur d'oxygène (12, 15),
**caractérisé en ce que**
un capteur d'oxygène galvanique (12) ou un capteur d'oxygène paramagnétique (15) peut être utilisé sélectivement dans le logement (5), dans lequel les deux capteurs d'oxygène ne s'adaptent pas conjointement dans le logement,
dans lequel un premier raccordement (8) associé au respirateur et deux seconds raccordements (9) sont prévus dans la zone du logement (5) pour le raccordement électrique du capteur d'oxygène (12, 15) au respirateur (1), dans lequel l'un second raccordement (9) est prévu pour le raccordement à un capteur d'oxygène galvanique (12) et l'autre second raccordement (9) pour le raccordement à un capteur d'oxygène paramagnétique (15).

2. Respirateur (1) selon la revendication 1,
**caractérisé en ce que**
le capteur d'oxygène (12, 15) peut être fixé au moyen d'une pièce de fixation (14) dans le logement (5).

3. Respirateur (1) selon la revendication 2,
**caractérisé en ce que**
la pièce de fixation (14) présente un filetage (17) pour le raccordement à un capteur d'oxygène galvanique (12).

4. Respirateur selon la revendication 2 ou 3,
**caractérisé en ce que**
la pièce de fixation (14) présente un certain nombre d'alésages (18) pour le raccordement à un capteur d'oxygène paramagnétique (15) au moyen d'un certain nombre de vis (19).

5. Respirateur (1) selon l'une quelconque des revendications 1 à 4,
**caractérisé en ce que**
les seconds raccordements (9) sont disposés dans la zone du logement (5) de telle sorte que dans le cas d'un capteur galvanique (12) utilisé dans le logement (5) celui-ci peut être raccordé uniquement à l'un second raccordement (9), tandis que dans le cas d'un capteur d'oxygène paramagnétique (15) utilisé dans le logement (5) celui-ci peut être raccordé uniquement à l'autre second raccordement (9).

6. Respirateur (1) selon l'une quelconque des revendications 1 à 5,
**caractérisé en ce que**
le capteur d'oxygène (12, 15) peut être raccordé au respirateur (1) par le biais d'un câble Y (7).

7. Respirateur (1) selon l'une quelconque des revendications 1 à 6,
**caractérisé en ce que**
une unité de commande (6) du respirateur (1) reconnaît quel capteur d'oxygène (12, 15) est utilisé dans le logement (5).

8. Respirateur (1) selon l'une quelconque des revendications 1 à 7,
**caractérisé en ce que**
le capteur d'oxygène (12, 15) présente une unité électronique, au moyen de laquelle il peut communiquer avec une unité de commande (6) du respirateur (1).

9. Respirateur (1) selon l'une quelconque des revendications 1 à 8,
**caractérisé en ce que**
le logement (5) peut être verrouillé par un couvercle de verrouillage (11), lorsqu'aucun capteur d'oxygène (12, 15) n'est utilisé.

10. Respirateur (1) selon l'une quelconque des revendications 1 à 9,
**caractérisé en ce que**
le respirateur (1) est conçu aussi bien pour le fonctionnement mobile que stationnaire.
